# EUROPEAN PATENT APPLICATION

(11) **EP 1 094 074 A2**
(43) Date of publication of application: **25.04.2001**
(21) Application number: 00122243.9
(22) Date of filing: 23.06.1993
(51) Int. Cl.: C07K 14/655, A61K 38/31, A61K 51/08, G01N 33/68

(54) **Radioactively-labeled somatostatin derived peptides for imaging and therapeutic uses**

(30) Priority: 23.06.1992 US 902935
(62) Divisional of application: 93918129.3
(71) Applicant: Diatide, Inc., Londonderry, NH 03053 (US)
(72) Inventor: Dean, Richard T., Bedford, NH 03110 (US); Lister-James, John, Bedford, NH 03110 (US)
(74) Representative: Dörries, Hans Ulrich, Dr.

(57) **Abstract**

This invention relates to therapeutic reagents and peptides, radiodiagnostic reagents and peptides, and methods for producing labeled radiodiagnostic agents. Specifically, the invention relates to peptide derivatives and analogs of somatostatin, and embodiments of such peptides labeled with technetium-99m (Tc-99m), as well as methods and kits for making, radiolabeling and using such peptides to image sites in a mammalian body. The invention also relatees to peptide derivatives and analogues of somatostantin labeled with rhenium-186 (¹⁸⁶Re) and rhenium-188 (¹⁸⁸Re), and methods and kits for making, radiolabeling and using such peptides therapeutically in a mammalian body.

## Description

### BACKGROUND OF THE INVENTION

### 1. Field of the Invention

This invention relates to therapeutic reagents and peptides, radiodiagnostic reagents and peptides, and methods for producing labeled radiodiagnostic and radiotherapeutic agents. Specifically, the invention relates to peptide derivatives and analogues of somatostatin, and embodiments of such peptides labeled with technetium-99m (Tc-99m), as well as methods and kits for making, radiolabeling and using such peptides to image sites in a mammalian body. The invention also relates to peptide derivatives and analogues of somatostatin labeled with rhenium-186 (¹⁸⁶Re) and rhenium-188 (¹⁸⁸Re), and methods and kits for making, radiolabeling and using such peptides therapeutically in a mammalian body.

### 2. Description of the Prior Art

Somatostatin is a tetradecapeptide that is endogenously produced by the hypothalamus and pancreas in humans and other mammals. The peptide has the formula: 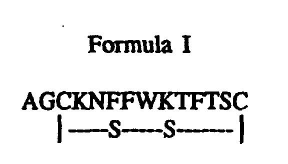 [Single letter abbreviations for amino acids can be found in G. Zubay, Biochemistry (2d ed.), 1988, (MacMillan Publishing: New York), p.33]. This peptide exerts a wide variety of biological effects *in vivo*. It is known to act physiologically on the central nervous system, the hypothalamus, the pancreas, and the gastrointestinal tract.

Somatostatin inhibits the release of insulin and glucagon from the pancreas, inhibits growth hormone release from the hypothalamus, and reduces gastric secretions. Thus, somatostatin has clinical and therapeutic applications for the alleviation of a number of ailments and diseases, both in humans and other animals. Native somatostatin is of limited utility, however, due to its short half-life *in vivo,* where it is rapidly degraded by peptidases. For this reason, somatostatin analogues having improved *in vivo* stability have been developed in the prior art.

Freidinger, U.S. Patent No. 4,235,886 disclose cyclic hexapeptide somatostatin analogues useful in the treatment of a number of diseases in humans.

Freidinger, U.S. Patent No. 4,611,054 disclose cyclic hexapeptide somatostatin analogues useful in the treatment of a number of diseases in humans.

Nutt, U.S. Patent No. 4,612,366 disclose cyclic hexapeptide somatostatin analogues useful in the treatment of a number of diseases in humans.

Coy *et al*., U.S. Patent No. 4,853,371 disclose synthetic octapeptide somatostatin analogues.

Coy and Murphy, U.S. Patent No. 4,871,717 disclose synthetic heptapeptide somatostatin analogues.

Coy and Murphy, U.S. Patent No. 4,485,101 disclose synthetic dodecapeptide somatostatin analogues.

Coy *et al*., U.S. Patent No. 4,904,642 disclose synthetic octapeptide somatostatin analogues.

Brady, European Patent Application No. 83111747.8 discloses dicyclic hexapeptide somatostatin analogues useful in the treatment of a number of human diseases.

Bauer *et al*., European Patent Application No. 85810617.2 disclose somatostatin derivatives useful in the treatment of a number of human diseases.

Eck and Moreau, European Patent Application No. 90302760.5 disclose therapeutic octapeptide somatostatin analogues.

Cox, International Patent Application No. PCT/US92/04559 discloses radiolabeled somatostatin derivatives containing two cysteine residues.

Somatostatin exerts it effects by binding to specific receptors expressed at the cell surface of cells comprising the central nervous system, the hypothalamus, the pancreas, and the gastrointestinal tract. These high-affinity somatostatin binding sites have been found to be abundantly expressed at the cell surface of most endocrine-active tumors arising from these tissues. Expression of high-affinity binding sites for somatostatin is a marker for these tumor cells, and specific binding with somatostatin can be exploited to locate and identify tumor cells *in vivo*.

Methods for radiolabeling somatostatin analogues that have been modified so as to contain a tyrosine amino acid (Tyr or Y) are known in the prior art.

Albert *et al*., UK Patent Application 8927255.3 disclose radioimaging using somatostatin derivatives such as octreotide labeled with ¹²³I.

Bakker *et al*., J. Nucl. Med. 31: 1501-1509 (1990) describe radioactive iodination of a somatostatin analog and its usefulness in detecting tumors *in vivo*.

Bakker *et al*., J. Nucl. Med. 32: 1184-1189 (1991) teach the usefulness of radiolabeled somatostatin for radioimaging *in vivo*.

Alternatively, methods for radiolabeling somatostatin by covalently modifying the peptide to contain a radionuclide-chelating group have been disclosed in the prior art.

Albert *et al*., UK Patent Application 8927255.3 disclose radioimaging using somatostatin derivatives such as octreotide labeled with ¹¹¹In *via* a chelating group bound to the amino-terminus.

Albert *et al*., European Patent Application No. WO 91/01144 disclose radioimaging using radiolabeled peptides related to growth factors, hormones, interferons and cytokines and comprised of a specific recognition peptide covalently linked to a radionuclide chelating group.

Albert *et al*., European Patent Application No. 92810381.1 disclose somatostatin peptides having amino-terminally linked chelators.

Bodgen and Moreau, International Patent Application Serial No. PCT/US92/01027 disclose compositions and methods for treating proliferative skin disease.

Faglia *et al*., 1991, J. Clin. Endocrinol. Metab. 73: 850-856 describe the detection of somatostatin receptors in patients.

Kwekkeboom *et al*., J. Nucl. Med. 32: 981 (1991) Abstract #305 relates to radiolabeling somatostatin analogues with ¹¹¹In.

Albert *et al*., Abstract LM10, 12th American Peptide Symposium: 1991 describe uses for ¹¹¹In-labeled diethylene-triaminopentaacetic acid-derivatized somatostatin analogues.

Krenning *et al*., 1992, J. Nucl. Med. 33: 652-658 describe clinical scintigraphy using [¹¹¹In][DTPA]octreotide.

These methods can be readily adapted to enable detection of tumor cells *in vivo*, by radioimaging, based on the expression of high affinity binding sites for somatostatin on tumor cells. Radionuclides which emit high energy gamma radiation can be readily detected by scintigraphy after injection into a human or an animal. A variety of radionuclides are known to be useful for radioimaging, including ⁶⁷Ga, ⁶⁸Ga, ^{99m}Tc (Tc-99m), ¹¹¹In, ¹²³I or ¹²⁵I. The sensitivity of imaging methods using radioactively-labeled peptides is much higher than other techniques known in the art, since the specific binding of the radioactive peptide concentrates the radioactive signal over the cells of interest, for example, tumor cells. This is particularly important for endocrine-active gastrointestinal tumors, which are usually small, slow-growing and difficult to detect by conventional methods. Labeling with technetium-99m (Tc-99m) is advantageous because the nuclear and radioactive properties of this isotope make it an ideal scintigraphic imaging agent. Tc-99m has a single photon energy of 140 keV and a radioactive half-life of about 6 hours, and is readily available from a ⁹⁹Mo-^{99m}Tc generator. Other radionuclides have effective half-lives which are much longer (*for example*, ¹¹¹In, which has a half-life of 60-70 h) or are toxic (for example, ¹²⁵I). Although Tc-99m is an ideal radiolabeling reagent, it has not been widely used in the art prior to the present invention [*see, for example*, Lamberts, J. Nucl. Med. 32: 1189-1191 (1991)].

Somatostatin and radiolabeled somatostatin analogues can also be used therapeutically. For these applications, the rhenium isotopes ¹⁸⁶Re and ¹⁸⁸Re are particularly advantageous.

Taylor *et al*., U.S. Patent No. 5,073,541 disclose a method of treating small cell lung cancer.

Coy and Murphy, International Patent Application Serial No. PCT/US90/07074 disclose somatostatin analogues for therapeutic uses.

Schally *et al*., European Patent Application Serial No. EPA 911048445.2 disclose cyclic peptides for therapeutic use.

Bomanji *et al*., 1992, J. Nucl. Med. 33: 1121-1124 describe the use of iodinated (Tyr-3) octreotide for imaging metastatic carcinoid tumors.

The use of chelating agents for radiolabeling proteins are known in the prior art, and methods for labeling peptides Tc-99m are disclosed in co-pending U.S. Patent Applications Serial Nos. 07/653,012, 07/757,470, 07/807,062, 07/851,074, 07/871,282, 07/886,752, 07/893,981, 07/955,466, 07/977,628, 08/019,864, 08/044,825 and 08/ , and PCT International Applications PCT/US92/00757, PCT/US92/10716, PCT/US93/02320, PCT/US93/03687, PCT/US93/04794, and PCT/US93/ , which are hereby incorporated by reference.

Fritzberg, U.S. Patent No. 4,444,690 describes a series of technetium-chelating agents based on 2,3-bis(mercaptoacetamido) propanoate.

Gansow *et al*., U.S. Patent No. 4,472,509 teach methods of manufacturing and purifying Tc-99m chelate-conjugated monoclonal antibodies.

Reno and Bottino, European Patent Application 87300426.1 disclose radiolabeling antibodies with Tc-99m.

Pak *et al*., European Patent Application No. WO 88/07382 disclose a method for labeling antibodies with Tc-99m.

Rhodes, Sem. Nucl. Med. 4: 281-293 (1974) teach the labeling of human serum albumin with technetium-99m.

Khaw *et al*., J. Nucl. Med. 23: 1011-1019 (1982) disclose methods for labeling biologically active macromolecules with Tc-99m.

Byrne and Tolman, *supra*, disclose a bifunctional thiolactone chelating agent for coupling Tc-99m to biological molecules.

Cox *et al*., Abstract, 7th International Symposium on Radiopharmacology, p. 16, 1991, disclose the use of, Tc-99m-, ¹³¹I- and ¹¹¹In-labeled somatostatin analogues in radiolocalization of endocrine tumors *in vivo* by scintigraphy.

Methods for directly labeling somatostatin, derivatives of somatostatin, analogues of somatostatin or peptides that bind to the somatostatin receptor and contain at least 2 cysteine residues that form a disulfide or wherein the disulfide is reduced to the sulfhydryl form, are disclosed in co-pending U.S. Patent Application Serial No. 07/807,062, now U.S. Patent No. 5,225,180, issued July 6, 1993 which is hereby incorporated by reference.

There remains a need for synthetic (to make routine manufacture practicable and to ease regulatory acceptance) somatostatin analogues having increased *in vivo* stability, to be used therapeutically and as scintigraphic agents when radiolabeled with Tc-99m for use in imaging tumors *in vivo*. Small synthetic somatostatin analogues are provided by this invention that specifically fulfill this need.

### SUMMARY OF THE INVENTION

The present invention provides somatostatin analogues that are peptide reagents for therapeutic and scintigraphic imaging applications. Specifically, the invention provides peptide reagents for preparing scintigraphic imaging agents that are technetium-99m (Tc-99m) labeled. The scintigraphic imaging agents of the invention are comprised of a peptide that is a somatostatin analogue covalently linked to a Tc-99m binding moiety and labeled with Tc-99m. In addition, the invention provides somatostatin analogues that are useful therapeutically, such analogues being radiolabeled with ¹⁸⁶Re and ¹⁸⁸Re.

The somatostatin analogues provided by the invention are somatostatin-receptor binding peptides having the following formula: 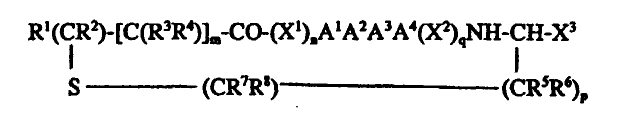 where R¹ and R² are independently H, lower alkyl or substituted alkyl, aryl or substituted aryl; R³ and R⁴ are each independently H, lower alkyl or substituted alkyl, aryl or substituted aryl, or either R³ or R⁴ are N(R¹⁰)₂, where each R¹⁰ is independently H, lower alkyl or a peptide sequence of no more than 10 amino acids, and m is an integer between 0 and 3; X¹ and X² are each independently a 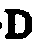 - or 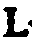 - amino acid, and n and q are independently either 0 or 1; A¹ is 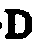 - or 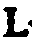 -Phe or 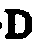 - or 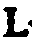 -Tyr or 2-naphthylalanine (Nal) or substituted derivatives thereof; A² is 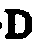 - or 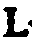 -Trp or substituted derivatives thereof; A³ is 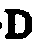 - or 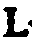 -Lys or homolysine (Hly), 4-amino-cyclohexylalanine (Achxa), 4-aminomethylphenylalanine (Amf), S-(2-aminoethyl)cysteine (Aec), S-(3-aminopropyl)cysteine (Apc), O-(2-aminoethyl) serine (Aes), O-(3-aminopropyl)serine (Aps) or substituted derivatives thereof; A⁴ is Thr, Ser, Val, Phe, Leu, Ile or 2-amino-isobutyric acid (Aib), 2-aminobutyric acid (Abu), norvaline (Nva), or norleucine (Nle), most preferably Thr or Val; X³ is H, -COOR⁹, -CH₂OH, CH₂COOR⁹, or -CON(R⁹)₂, where each R⁹ is independently H, lower linear or cyclic alkyl or substituted derivatives thereof, or a peptide having an amino acid sequence of no more than 10 residues; R⁵ and R⁶ are each independently H or lower alkyl and p is either 0, 1 or 2; and R⁷ and R⁸ are independently H, lower alkyl or substituted lower alkyl, or either R⁷ and R⁸ are -COOH or a derivative thereof. In a preferred embodiment, A¹ is Phe or Tyr, A² is Trp or most preferably 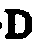 -Trp, A³ is Lys and A⁴ is Thr or Val.

In a first aspect of the present invention are provided peptide reagents that are somatostatin analogues as described herein having increased *in vivo* stability compared with native somatostatin, and that are therapeutically useful in the alleviation of diseases or other ailments in humans or other animals.

The invention also provides pharmaceutical compositions comprising the somatostatin receptor-binding peptides of the invention in a pharmaceutically acceptable carrier.

The invention also provides a method for alleviating somatostatin-related diseases in animals, preferably humans, comprising administering a therapeutically effective amount of the somatostatin analogues of the invention to the animal. In preferred embodiments, the amount of the somatostatin analogue administered is from about 0.1 to about 50 mg/kg body weight/day.

Another aspect of the present invention provides reagents for preparing scintigraphic imaging agents, each reagent comprising a peptide that is somatostatin analogue and is covalently linked to a Tc-99m binding moiety.

It is an advantage of the somatostatin analogues provided by this invention that the thioether linkage contained therein is stable under the conditions of Tc-99m conjugation to the covalently linked Tc-99m binding moiety. In contrast, Tc-99m conjugation to a Tc-99m binding moiety covalently linked to native somatostatin, or to a somatostatin analogue having a disulfide bond, can result in reduction of the disulfide accompanied by a loss of biological activity. Such loss of biological activity can also occur *in vivo* using native somatostatin, or to any somatostatin analogue having a disulfide bond. The present invention is not subject to similar losses in biological activity *in vivo* because the thioether linkage in each of the somatostatin analogues of the invention is a stable covalent bond.

It is another advantage of the somatostatin analogues provided by this invention that the covalent linkage between the amino terminus and the cysteine protecting moiety acts to protect the peptide from degradation by exopepetidases.

A first aspect of the reagents provided by the invention for preparing scintigraphic imaging agents of the invention are reagents, each comprised of a peptide that is a somatostatin analogue that is covalently linked to a Tc-99m binding moiety having formula:

C(pgp)^{S}-(aa)-C(pgp)^{S}

wherein (pgp)^{S} is H or a thiol protecting group and (aa) is an amino acid. In a preferred embodiment, the amino acid is glycine.

In a second embodiment, the invention provides peptide reagents capable of being Tc-99m labeled for imaging sites within a mammalian body, each comprising a somatostatin analogue that is covalently linked to a Tc-99m binding moiety of of formula:

A-CZ(B)-[C(R'R'')]ₙ-X II.

wherein A is H, HOOC, H₂NOC, (peptide)-NHOC, (peptide)-OOC or R''''; B is H, SH or -NHR''', -N(R''')-(peptide) or R''''; X is SH or -NHR''', -N(R''')-(peptide) or R''''; R', R'', R''' and R'''' are independently H or straight or branched chain or cyclic lower alkyl; n is 0, 1 or 2; and: (1) where B is -NHR''' or -N(R''')-(peptide), X is SH and n is 1 or 2; (2) where X is -NHR''' or -N(R''')-(peptide), B is SH and n is 1 or 2; (3) where B is H or R'''', A is HOOC, H₂NOC, (peptide)-NHOC, (peptide)-OOC, X is SH and n is 0 or 1; (4) where A is H or R'''', then where B is SH, X is -NHR''' or -N(R''')-(peptide) and where X is SH, B is -NHR''' or -N(R''')-(peptide); (5) where X is H or R'''', A is HOOC, H₂NOC, (peptide)-NHOC, (peptide)-OOC and B is SH; (6) where Z is methyl, X is methyl, A is HOOC, H₂NOC, (peptide)-NHOC, (peptide)-OOC and B is SH and n is 0; and (7) where Z is SH and X is SH, n is not 0; and wherein the thiol moiety is in the reduced form.

In another embodiment, the invention provides peptide reagents capable of being labeled with Tc-99m for imaging sites within a mammalian body, each comprising a somatostatin analogue that is covalently linked to a Tc-99m binding moiety of formula: 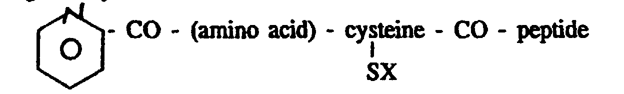 [for purposes of this invention, radiolabel-binding moieties having this structure will be referred to as picolinic acid (Pic)-based moieties]
or 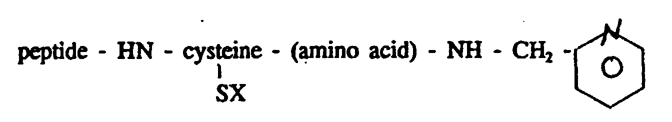 wherein X is H or a protecting group; (amino acid) is any amino acid and the radiolabel-binding moiety is covalently linked to the peptide. For purposes of this invention, radiolabel-binding moieties having this structure will be referred to as picolylamine (Pica)-based moieties. In a preferred embodiment, the amino acid is glycine and X is an acetamidomethyl protecting group.

Yet another embodiment of the invention provides peptide reagents capable of being labeled with Tc-99m for imaging sites within a mammalian body, each comprising a somatostatin analogue that is covalently linked to a Tc-99m binding moiety that is a bisamino bisthiol Tc-99m binding moiety. The bisamino bisthiol Tc-99m binding moiety in this embodiment of the invention has the formula: 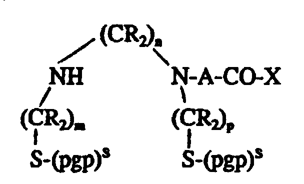 wherein each R can be independently H, CH₃ or C₂H₅; each (pgp)^{S} can be independently a thiol protecting group or H; m, n and p are independently 2 or 3; A is linear or cyclic lower alkyl, aryl, heterocyclyl, combinations or substituted derivatives thereof; and X is peptide; or 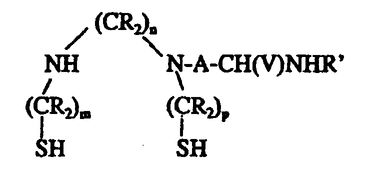 wherein each R is independently H, CH₃ or C₂H₅; m, n and p are independently 2 or 3; A is linear or cyclic lower alkyl, aryl, heterocyclyl, combinations or substituted derivatives thereof; V is H or CO-peptide; R 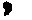 is H or peptide; provided that when V is H, R 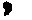 is peptide and when R 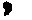 is H, V is peptide. For purposes of this invention, radiolabel-binding moieties having these structures will be referred to as "BAT" moieties.

The invention also comprises scintigraphic imaging agents that are complexes of the peptide reagents of the invention with Tc-99m and methods for radiolabeling the peptide reagents of the invention with Tc-99m. Radiolabeled complexes provided by the invention are formed by reacting the peptide reagents of the invention with Tc-99m in the presence of a reducing agent. Preferred reducing agents include but are not limited to dithionite ion, stannous ion and ferrous ion. Complexes of the invention are also formed by labeling the peptide reagents of the invention with Tc-99m by ligand exchange of a prereduced Tc-99m complex as provided herein.

The invention also provides kits for preparing scintigraphic imaging agents that are the peptide reagents of the invention radiolabeled with Tc-99m. Kits for labeling the peptide reagents of the invention with Tc-99m are comprised of a sealed vial containing a predetermined quantity of a peptide reagent of the invention and a sufficient amount of reducing agent to label the peptide with Tc-99m.

This invention provides methods for preparing peptide reagents of the invention by chemical synthesis *in vitro*. In a preferred embodiment, peptides are synthesized by solid phase peptide synthesis.

This invention provides methods for using scintigraphic imaging agents that are Tc-99m labeled peptide reagents for imaging sites within a mammalian body by obtaining *in vivo* gamma scintigraphic images. These methods comprise administering an effective diagnostic amount of Tc-99m labeled peptide reagents of the invention and detecting the gamma radiation emitted by the Tc-99m label localized at the site within the mammalian body.

This invention provides reagents for preparing a radiolabled somatostatin receptor-binding agent comprising the somatostatin receptor-binding peptides of the invention covalently linked to a radiolabel-binding moiety. In a preferred embodiment, the reagent is radioactively labeled with Tc-99m. In another preferred embodiment, the reagent is radioactively labeled with ¹⁸⁶Re or ¹⁸⁸Re.

The invention also provides methods for alleviating somatostatin-related diseases in animals, preferably humans, comprising administering a therapeutically effective amount of the radiolabeled somatostatin-binding peptide reagents of the invention to the animal. In preferred embodiments, the reagent is radioactively labeled with ¹⁸⁶Re or ¹⁸⁸Re.

The reagents of the invention may also be comprised of a polyvalent linking moiety. Polyvalent linking moieties of the invention are comprised of at least 2 identical linker functional groups capable of covalently bonding to somatostatin analogue peptides or Tc-99m binding moieties. Preferred linker functional groups are primary or secondary amines, hydroxyl groups, carboxylic acid groups or thiol-reactive groups. In preferred embodiments, the polyvalent linking moieties are comprised of *bis*-succinimidylmethylether (BSME), 4-(2,2-dimethylacetyl)benzoic acid (DMBA), *N-*[2*-*(*N',N'-bis*(2-succinimidoethyl)aminoethyl)]-*N*⁶*,N*⁹*-bis*(2-methyl-2-mercaptopropyl)-6,9-diazanonanamide (BAT-BS), *tris*(succinimidylethyl)amine (TSEA), *bis*-succinimidohexane (BSH), 4-(O-CH₂CO-Gly-Gly-Cys.amide)acetophenone (ETAC) or a derivative thereof.

Specific preferred embodiments of the present invention will become evident from the following more detailed description of certain preferred embodiments and the claims.

### DETAILED DESCRIPTION OF THE INVENTION

The present invention provides peptide reagents for preparing radiolabeled imaging agents for imaging site within a mammalian body. The peptide reagents of the invention each comprise a somatostatin analogue that is covalently linked to a Tc-99m binding moiety. The invention also provides somatostatin analogues having an increased *in vivo* stability and that are useful for alleviating diseases or other ailments in humans or other animals.

The invention provides a method for using the somatostatin analogues of the invention to alleviate diseases or other ailments in animals, preferably humans. These diseases and ailments include but are not limited to diabetes and diabetes-related retinopathy, cirrhosis of the liver and hepatitis infection, bleeding ulcers and other gastrointestinal bleeding, pancreatitis, central nervous system disorders, endocrine disorders, Alzheimer's disease, acromegaly and other diseases and disorders related to the production of inappropriate levels of growth hormone *in vivo*, and cancer, particularly those cancers whose growth is dependent or influenced by growth hormone production. Dosages of the somatostatin analogues provided by the invention may be the same as those dosages of native somatostatin routinely used for treatment of the above or other diseases, or less of the compounds of the invention may be administered due to their longer *in vivo* half-life.

Labeling with Tc-99m is an advantage of the present invention because the nuclear and radioactive properties of this isotope make it an ideal scintigraphic imaging agent. This isotope has a single photon energy of 140 keV and a radioactive half-life of about 6 hours, and is readily available from a ⁹⁹Mo-^{99m}Tc generator. Other radionuclides known in the prior art have effective half-lives which are much longer (*for example*, ¹¹¹In, which has a half-life of 67.4 h) or are toxic (*for example*, ¹²⁵I).

Radiotherapeutic embodiments of the invention, on the other hand, are advantageously labeled with ¹⁸⁶Re or ¹⁸⁸Re. Such embodiments are useful in the treatment of somatostatin-related diseases or other ailments in animals, preferably humans, including but not limited to cancer and other diseases characterized by the growth of malignant or benign tumors capable of binding somatostatin or somatostatin analogues *via* the expression of somatostatin receptors on the cell surface of cells comprising such tumors.

In the Tc-99m binding moieties and peptides covalently linked to such moieties that contain a thiol covalently linked to a thiol protecting groups [(pgp)^{S}] provided by the invention, the thiol-protecting groups may be the same or different and may be but are not limited to:
-CH₂-aryl (aryl is phenyl or alkyl or alkyloxy substituted phenyl);
-CH-(aryl)₂, (aryl is phenyl or alkyl or alkyloxy substituted phenyl);
-C-(aryl)₃, (aryl is phenyl or alkyl or alkyloxy substituted phenyl);
-CH₂-(4-methoxyphenyl);
-CH-(4-pyridyl)(phenyl)₂;
-C(CH₃)₃
-9-phenylfluorenyl;
-CH₂NHCOR (R is unsubstituted or substituted alkyl or aryl);
-CH₂-NHCOOR (R is unsubstituted or substituted alkyl or aryl);
-CONHR (R is unsubstituted or substituted alkyl or aryl);
-CH₂-S-CH₂-phenyl

Preferred protecting groups have the formula -CH₂-NHCOR wherein R is a lower alkyl having 1 and 8 carbon atoms, phenyl or phenyl-substituted with lower alkyl, hydroxyl, lower alkoxy, carboxy, or lower alkoxycarbonyl. The most preferred protecting group is an acetamidomethyl group.

Each somatostatin receptor-binding peptide-containing embodiment of the invention is comprised of a sequence of amino acids. The term amino acid as used in this invention is intended to include all 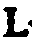 - and 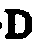 - amino acids, naturally occurring and otherwise. Reagents comprising somatostatin receptor-binding peptides provided by the invention include but are not limited to the following illustrative examples of the peptide embodiments of the invention:
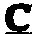 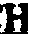 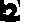 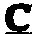 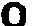 .YW_{D}KTCTC_{Acm}GC_{Acm}.amide
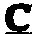 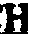 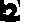 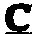 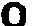 .YW_{D}KTC
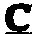 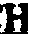 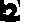 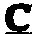 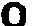 .YW_{D}KTC.amide
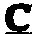 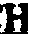 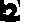 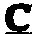 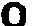 .YW_{D}KTCT
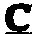 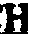 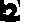 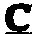 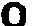 .YW_{D}KTCT( 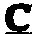 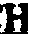 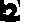 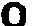 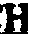 )
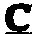 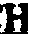 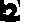 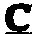 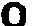 .YW_{D}KTCTGGC_{Mob}.amide
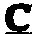 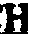 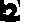 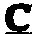 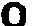 .FFW_{D}KTFC
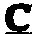 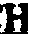 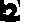 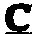 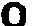 .FFW_{D}KTFC.[BAM]
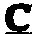 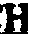 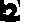 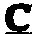 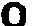 .FFW_{D}KTFC.amide
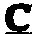 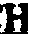 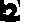 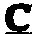 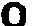 .FW_{D}KT.Hcy
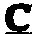 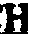 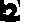 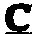 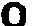 .FW_{D}KTC_{D}
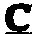 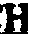 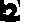 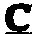 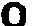 .FW_{D}KT.Hcy.amide
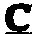 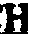 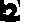 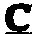 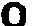 .FW_{D}KT.Pen
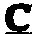 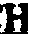 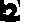 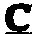 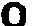 .NFFW_{D}KTFTC
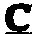 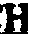 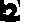 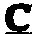 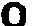 .FFW_{D}KTFCC_{Acm}GC_{Acm}.amide
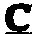 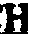 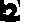 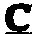 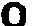 .FFW_{D}KTF.Hcy
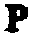 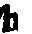 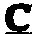 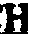 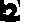 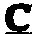 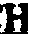 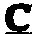 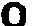 .YW_{D}KTC
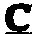 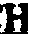 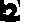 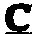 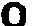 .YW_{D}KT.Hhc
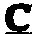 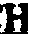 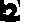 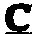 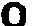 .YW_{D}KT.Hhc.amide
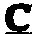 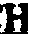 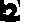 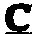 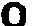 .FFW_{D}KTF.Hhc
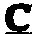 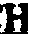 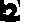 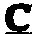 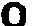 .FYW_{D}KTFC

As used herein, the following amino acids and amino acid analogues are intended to be represented by the following abbreviations: Hcy is homocysteine, prepared by alkaline hydrolysis of 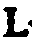 -homocysteine lactone; Hhc is homohomocysteine; Pen is penicillamine; Mob is the sulfhydrul protecting group 4-methoxybenzyl; Acm is the sulfhydryl protecting group acetamidomethyl; [BAM] is (*N*¹,*N*⁴-*bis*(2-mercapto-2-methylpropyl)-1,4,10-triazadecane; Aib is aminoisobutyric acid; Nal is 2-naphthylalanine; Ain is 2-aminoindanoic acid; Hly is homolysine; Achxa is 4-amino-cyclohexylalanine; Amf is 4-aminomethylphenylalanine; Aec is S-(2-aminoethyl)cysteine; Apc is S-(3-aminopropyl)cysteine; Aes is O-(2-aminoethyl)serine; Aps is O-(3-aminopropyl)serine; Abu is 2-aminobutyric acid; Nva is norvaline; Aca is 6-aminocaproic acid; and Nle is norleucine. All naturally-occurring amino acids are abbreviated using standard abbreviations (which can be found in G. Zubay, *Biochemistry* (2d. ed.), 1988 (MacMillen Publishing: New York) p.33. T( 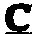 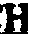 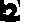 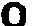 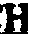 ) represents a threoninol residue, wherein the carboxyl group of the amino acid is reduced to a primary alcohol, incorporated into the peptide using the procedure of Neugebauer *et al.* (1990, Peptides: Proceedings of the 11th American Peptide Symposium, pp. 1020-21).

It will also be understood by those with skill in the art that the convention of representing by underlining a covalent bond between the sidechain sulfur atom of a cysteine residue or derivative thereof and a protecting group or other residue is used herein.

Somatostatin analogue peptides of the present invention can be chemically synthesized *in vitro*. Peptides of the present invention can generally advantageously be prepared on a peptide synthesizer. The peptides of this invention can be synthesized wherein the radiolabel-binding moiety is covalently linked to the peptide during chemical synthesis *in vitro*, using techniques well known to those with skill in the art. Such peptides covalently-linked to the radiolabel-binding moiety during synthesis are advantageous because specific sites of covalent linkage can be determined.

Radiolabel binding moieties of the invention may be introduced into the target somatostatin analogue peptides during peptide synthesis. For embodiments comprising picolinic acid [(Pic-); *e.g.*, Pic-Gly-Cys(protecting group)-], the radiolabel-binding moiety can be synthesized as the last (i.e., amino-terminal) residue in the synthesis. In addition, the picolinic acid-containing radiolabel-binding moiety may be covalently linked to the ε-amino group of lysine to give, for example, αN(Fmoc)-Lys-εN[Pic-Gly-Cys(protecting group)], which may be incorporated at any appropriate position in the peptide chain. This sequence is particularly advantageous as it affords an easy mode of incorporation into the target somatostatin analogue peptide.

Similarly, the picolylamine (Pica)-containing radiolabel-binding moiety [-Cys(protecting group)-Gly-Pica] can be prepared during peptide synthesis by including the sequence [-Cys(protecting group)-Gly-] at the carboxyl terminus of the peptide chain. Following cleavage of the peptide from the resin the carboxyl terminus of the peptide is activated and coupled to picolylamine. This synthetic route requires that reactive side-chain functionalities remain masked (protected) and do not react during the conjugation of the picolylamine.

This invention also provides small synthetic peptides that are somatostatin analogues and incorporate bisamine bisthiol (BAT) chelators that may be labeled with Tc-99m.

This invention provides for the incorporation of these chelators into virtually any position in the peptide, *via* covalently linkage to any appropriate functional group of the peptide, except that the chelating moieties of the invention are not covalently linked to functional groups comprising the amino acid side chains of the amino acids A¹, A², A³ or A⁴.

In forming a complex of radioactive technetium with the reagents of this invention, the technetium complex, preferably a salt of Tc-99m pertechnetate, is reacted with the reagent in the presence of a reducing agent. Preferred reducing agents are dithionite, stannous and ferrous ions; the most preferred reducing agent is stannous chloride. Means for preparing such complexes are conveniently provided in a kit form comprising a sealed vial containing a predetermined quantity of a reagent of the invention to be labeled and a sufficient amount of reducing agent to label the reagent with Tc-99m. Alternatively, the complex may be formed by reacting a reagent of this invention with a pre-formed labile complex of technetium and another compound known as a transfer ligand. This process is known as ligand exchange and is well known to those skilled in the art. The labile complex may be formed using such transfer ligands as tartrate, citrate, gluconate or mannitol, for example. Among the Tc-99m pertechnetate salts useful with the present invention are included the alkali metal salts such as the sodium salt, or ammonium salts or lower alkyl ammonium salts.

In a preferred embodiment of the invention, a kit for preparing technetium-labeled peptides is provided. An appropriate amount of the peptide reagent is introduced into a vial containing a reducing agent, such as stannous chloride, in an amount sufficient to label the peptide with Tc-99m. An appropriate amount of a transfer ligand as described (such as tartrate, citrate, gluconate or mannitol, for example) can also be included. The kit may also contain conventional pharmaceutical adjunct materials such as, for example, pharmaceutically acceptable salts to adjust the osmotic pressure, buffers, preservatives and the like. The components of the kit may be in liquid, frozen or dry form. In a preferred embodiment, kit components are provided in lyophilized form.

Radiolabeled imaging reagents according to the present invention may be prepared by the addition of an appropriate amount of Tc-99m or Tc-99m complex into the vials and reaction under conditions described in Example 2 hereinbelow.

Radioactively-labeled scintigraphic imaging agents provided by the present invention are provided having a suitable amount of radioactivity. In forming Tc-99m radioactive complexes, it is generally preferred to form radioactive complexes in solutions containing radioactivity at concentrations of from about 0.01 millicurie (mCi) to 100 mCi per mL.

The imaging reagents provided by the present invention can be used for visualizing organs such as the kidney for diagnosing disorders in these organs, and tumors, in particular gastrointestinal tumors, myelomas, small cell lung carcinoma and other APUDomas, endocrine tumors such as medullary thyroid carcinomas and pituitary tumors, brain tumors such as meningiomas and astrocytomas, and tumors of the prostate, breast, colon, and ovaries can also be imaged. In accordance with this invention, the Tc-99m labeled peptide reagents are administered in a single unit injectable dose. The Tc-99m labeled peptide reagents provided by the invention may be administered intravenously in any conventional medium for intravenous injection such as an aqueous saline medium, or in blood plasma medium. Generally, the unit dose to be administered has a radioactivity of about 0.01 mCi to about 100 mCi, preferably 1 mCi to 20 mCi. The solution to be injected at unit dosage is from about 0.01 mL to about 10 mL. After intravenous administration, imaging *in vivo* can take place in a matter of a few minutes. However, imaging can take place, if desired, in hours or even longer, after the radiolabeled peptide is injected into a patient. In most instances, a sufficient amount of the administered dose will accumulate in the area to be imaged within about 0.1 of an hour to permit the taking of scintiphotos. Any conventional method of scintigraphic imaging for diagnostic purposes can be utilized in accordance with this invention.

The somatostatin receptor-binding peptides of the invention may be used clinically to promote regression of certain types of tumors, particularly those that express somatostatin receptors. The somatostatin analogue peptides of the invention can also be used to reduce the hormonal hypersecretion that often accompanies certain cancers, such as the APUDomas. Peptides of the invention used as threapeutic agents may be administered by any appropriate route, including intravenous, intramuscular or by mouth, and in any acceptable pharmaceutical carrier, in doses ranging from about 0.1 to about 49 mg/kgbody weight/day.

This invention also provides peptides radiolabled with rhenium-186 or rhenium-188 that may be used for radiotherapy of certain tumors as described above. For this purpose, an amount of radioactive isotope from about 10mCi to about 200mCi may be administered *via* any suitable clinical route, preferably by intravenous injection.

The methods for making and labeling these compounds are more fully illustrated in the following Examples. These Examples illustrate certain aspects of the above-described method and advantageous results, and are shown by way of illustration and not limitation.

### EXAMPLE 1

### Solid Phase Peptide Synthesis

Solid phase peptide synthesis (SPPS) was carried out on a 0.25 millimole (mmole) scale using an Applied Biosystems Model 431A Peptide Synthesizer and using 9-fluorenylmethyloxycarbonyl (Fmoc) amino-terminus protection, coupling with dicyclohexylcarbodiimide/hydroxybenzotriazole or 2-(1H-benzotriazol-1-yl)-1,1,3,3-tetramethyluronium hexafluorophosphate/hydroxybenzotriazole (HBTU/HOBT), and using *p*-hydroxymethylphenoxy-methylpolystyrene (HMP) resin for carboxyl-terminus acids or Rink amide resin for carboxyl-terminus amides.

Where appropriate, the following amino acid derivatives were synthesized. Homocysteine was prepared by alkaline hydrolysis of 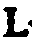 -homocysteine lactone. Threoninol residues, wherein the carboxyl group of the amino acid is reduced to a primary alcohol, are introduced into the peptides of the invention where appropriate using the procedure of Neugebauer *et al.* (1990, Peptides: Proceedings of the 11th American Peptide Symposium, pp. 1020-21). Fmoc.Hcy(Trt) and Fmoc.Pen(Trt) were prepared from the appropriate amino acids by tritylation with triphenylmethanol in TFA, followed by Fmoc derivitization as described by Atherton *et al.* (1989, Solid Phase Peptide Synthesis, IRL Press: Oxford). Fmoc.homohomo-cysteine(Trt) was prepared by reducing *N*,*N*-*bis*-Boc-glutamic acid-α-methyl ester with borane-THF, followed by mesylation and reaction with trityl-mercaptide, followed by removal of the Boc groups with BF₃OEt in acetic acid, and then Fmoc derivitization as described above. PhCH₂CHBrCOOH was prepared by treating phenylalanine (in a solution of water and TFA/ saturated with NaBr) with sodium nitrate, followed by distillation to recover the pure product.

Where appropriate, 2-chloroacetyl, 2-bromoacetyl and 2-bromo-3-phenylproprionyl groups were introduced either by using the appropriate 2-halo acid as the last residue coupled during SPPS, or by treating the N-terminus free amino acid peptide bound to the resin with either 2-halo acid/diisopropylcarbodiimide/*N*-hydroxysuccinimide/NMP or 2-halo acid anhydride/diisopropylethylamine/NMP.

Where appropriate, HPLC-purified 2-haloacylated peptides were cyclized by stirring an 0.1-1.0 mg/mL solution in phosphate or bicarbonate buffer or dilute ammonium hydroxide (pH 8.0), optionally containing 0.5-1.0 mM EDTA, or acetonitrile or THF for 1-48 h followed optionally by acidification with acetic acid, lyophilization and HPLC purification.

Where appropriate, [BAM](*N*¹,*N*⁴-*bis*(2-mercapto-2-methylpropyl)-1,4,10-triazadecane) was conjugated to the peptide by first activating the peptide carboxylate with a mixture of diisopropylcarbodiimide/ N-hydroxysuccinimide or HBTU/HOBt in DMF, NMP or methylene chloride, followed by coupling in the presence of diisopropylethylamine. After coupling, the conjugates were deprotected as described above.

Where appropriate, BSME adducts were prepared by reacting single thiol-containing peptides (5 to 50 mg/mL in DMF buffered to pH 7 with N-methyl-morpholine or N-ethyl-morpholine, or 50mM sodium phosphate buffer, pH 7-8, optionally containing 0.5mM EDTA or DMF or THF or acetonitrile) with 0.5 molar equivalents of BMME (*bis*-maleimidomethylether) pre-dissolved in acetonitrile at room temperature for approximately 1-18 hours. The solution was concentrated and the product was purified by HPLC.

Where appropriate, TSEA adducts were prepared by reacting single thiol-containing peptide (at concentrations of 10 to 100 mg/mL peptide in DMF buffered to pH 7 with N-methyl-morpholine or N-ethyl-morpholine, or 5 to 50 mg/mL peptide in 50mM sodium phosphate, pH 7-8, optionally containing 0.5mM EDTA or DMF or THF or acetonitrile) with 0.33 molar equivalents of TMEA (*tris*(2-maleimidoethyl)amine) pre-dissolved in acetonitrile or DMF, with or without 1 molar equivalent of triethanolamine, at room temperature for approximately 1-18h. Such reaction mixtures containing adducts were concentrated and the adducts were then purified using HPLC.

Where appropriate, BAT-BS (*N*-[2-(*N',N'-bis*(2-succinimidoethyl) aminoethyl)]-*N*⁶,*N*⁹-*bis*(2-methyl-2-mercaptopropyl)-6,9-diazanonanamide)adducts were prepared by reacting single thiol-containing peptide (at concentrations of 2 to 50 mg/mL peptide in DMF buffered to pH 7 with N-methyl-morpholine or N-ethyl-morpholine, or in 50mM sodium phosphate (pH 7-8), optionally containing 0.5mM EDTA or DMF or THF or acetonitrile) with 0.5 molar equivalents of BAT-BM (*N*-[2-(*N',N'-bis*(2-maleimidoethyl)aminoethyl)]-*N*⁹-(*t*-butoxycarbonyl)-*N*⁶,*N*⁹-*bis*(2-methyl-2-triphenylmethylthiopropyl)-6,9-diazanonanamide) pre-dissolved in acetonitrile or THF, at room temperature for approximately 1-18h. The solution was then evaporated to dryness and [BAT-BS]-peptide conjugates deprotected by treatment with 10mL TFA and 0.2mL triethylsilane for 1h. The solution was concentrated, the product adducts precipitated with ether, and then purified by HPLC.

Resin-bound products were routinely cleaved using a solution of trifluoroacetic acid or trifluoroacetic acid and methylene chloride, or optionally a solution of trifluoroacetic acid, water, thioanisole, ethanedithiol, and triethylsilane, prepared in ratios of 100 : 5 : 5 : 2.5 : 2 for 0.5 - 3 h at room temperature. Crude peptides were purified by preparative high pressure liquid chromatography (HPLC) using a Waters Delta Pak C18 column and gradient elution using 0.1% trifluoroacetic acid (TFA) in water modified with acetonitrile. Acetonitrile was evaporated from the eluted fractions which were then lyophilized. The identity of each product was confirmed by fast atom bombardment mass spectroscopy (FABMS) or by electrospray mass spectroscopy (ESMS).

The following somatostatin analogues were synthesized as provided herein, and the products of such synthesis identified by FABMS (MH⁺ values in parentheses):
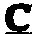 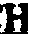 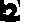 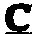 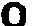 .YW_{D}KTCTC_{Acm}GC_{Acm}.amide (1246)
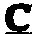 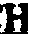 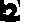 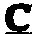 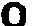 .YW_{D}KTC ( 740)
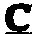 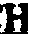 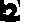 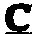 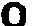 .YW_{D}KTC.amide ( 740)
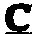 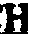 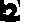 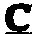 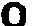 .YW_{D}KTCT ( 841)
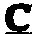 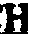 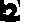 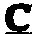 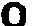 .YW_{D}KTCT( 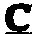 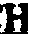 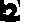 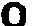 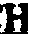 ) ( 828)
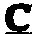 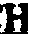 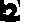 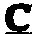 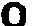 .YW_{D}KTCTGGC_{Mob}.amide (1178)
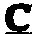 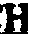 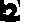 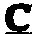 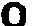 .FFW_{D}KTFC (1018)
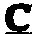 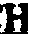 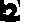 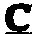 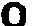 .FFW_{D}KTFC.[BAM] (1322)
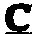 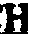 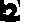 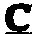 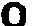 .FFW_{D}KTFC.amide (1017)
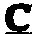 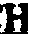 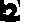 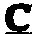 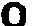 .FW_{D}KT.Hcy ( 738)
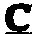 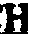 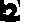 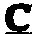 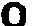 .FW_{D}KTC_{D} ( 724)
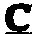 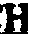 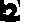 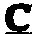 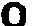 .FW_{D}KT.Hcy.amide ( 737)
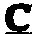 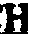 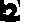 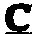 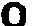 .FW_{D}KT.Pen ( 752)
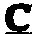 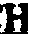 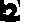 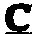 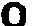 .NFFW_{D}KTFTC (1234)
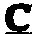 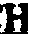 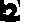 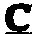 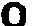 .FFW_{D}KTFCC_{Acm}GC_{Acm}.amide (1422)
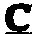 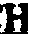 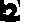 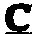 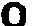 .FFW_{D}KTF.Hcy (1032)
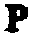 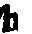 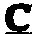 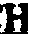 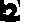 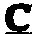 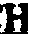 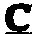 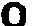 .YW_{D}KTC ( 830)
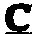 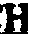 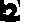 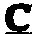 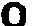 .YW_{D}KT.Hhc ( 769)
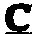 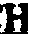 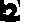 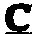 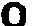 .YW_{D}KT.Hhc.amide ( 768)
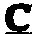 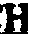 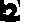 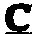 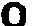 .FFW_{D}KTF.Hhc (1046)
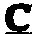 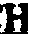 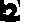 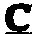 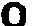 .FYW_{D}KTFC (1033)

### EXAMPLE 2

### A General Method for Radiolabeling with Tc-99m

0.1 mg of a peptide prepared as in Example 2 was dissolved in 0.1 mL of water or 50/50 ethanol/water or phosphate-buffered saline or 50 mM potassium phosphate buffer (pH = 5, 6 or 7.4). Tc-99m gluceptate was prepared by reconstituting a Glucoscan vial (E.I. DuPont de Nemours, Inc.) with 1.0 mL of Tc-99m sodium pertechnetate containing up to 200 mCi and allowed to stand for 15 minutes at room temperature. 25 µl of Tc-99m gluceptate was then added to the peptide and the reaction allowed to proceed at room temperature or at 100°C for 15-30 min and then filtered through a 0.2 µm filter.

The Tc-99m labeled peptide purity was determined by HPLC using the following conditions: a Waters Delta Pak RP-18, 5µ, 4.6mm x 220mm analytical column was loaded with each radiolabeled peptide, and the peptides eluted at a solvent flow rate equal to 1 mL/min. Gradient elution was performed beginning with 100% solvent A (0.1% CF₃COOH/H₂O) and ending with 1005 solvent B₉₀ (0.1% CF₃COOH/90% CH₃CN/H₂O) over the course of 10-20 min.

Radioactive components were detected using an in-line radiometric detector linked to an integrating recorder. Tc-99m gluceptate and Tc-99m sodium pertechnetate elute between 1 and 4 minutes under these conditions, whereas the Tc-99m labeled peptides eluted after a much greater amount of time, as illustrated in Table I below.

**TABLE I**

| Peptides | FABMS MH⁺ | Radiochemical Yield | HPLC Rₜ(min) |
|---|---|---|---|
| P389 | 1422 | 99%* | 15.1-16.9 |
| P428 | 1322 | 99%** | 18.8 |
| 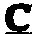 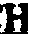 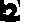 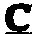 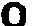 FFW_{D}KTFCC_{Acm}GC_{Acm}.amide = P389 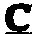 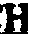 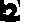 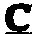 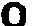 FFW_{D}KTFC.[BAM] = P428 | | | |

| | | | |
|---|---|---|---|
| * 1:1 ethanol:water, 100°C | | | |
| ** 1:1 ethanol:water, room temperature | | | |

### EXAMPLE 3

### Inhibition of Binding of [¹²⁵I-Tyr¹¹]somatostatin-14 to AR42J Rat Pancreatic Tumor Cell Membranes

The ability of various somatostatin analogues of the invention to bind to somatostatin receptors *in vitro* was demonstrated by assaying the ability of such analogues to inhibit binding of a radiolabeled somatostatin analogue to somatostatin receptor-containing cell membranes. The rat pancreatic tumor cell line AR42J which expresses the somatostatin receptor was cultured in Dulbecco's minimal essential media (DMEM) supplemented with 10% fetal bovine serum (FBS) and 8mM glutamine in a humdified 5% CO₂ atmosphere at 37°C in T-flasks. Harvested cells were homogenized in cold 50mM Tris-HCl buffer (pH 7.4) and the homogenate then centrifuged at 39,000*g* for 10min at 4°C. Pellets were washed once with buffer and then resuspended in an ice-cold solution of 10mM Tris-HCl (pH 7.4). Equal aliquots of this cell membrane preparation were incubated with [¹²⁵I-Tyr¹¹]somatostatin-14 (at a final concentration of 0.5nM and 750,000cpm/mL, at a specific activity of 2000Ci/mmol, Amersham, Arlington Heights, IL) and peptide at a final concentration of from 10⁻¹¹M to 10⁻⁶M in a solution of 50mM HEPES (pH 7.4) containing 1% bovine serum albumin (BSA), 5mM MgCl₂, Trasylol (200,000 International Units), bacitracin (0.02mg/mL) and phenylmethylsulfonylfluoride (0.02mg/mL) for 25min at 30°C. Using a filtration manifold, this mixture was filtered through a polyethyleneimine-washed GC/F filter (Whatman, Maidstone, England), and the residue remaining on the filter washed thrice with 5mL cold HEPES buffer. The filter and a sample of the filter washings were then counted in a gamma counter. To assess non-specific binding, the assay was performed in the presence of unlabeled somatostatin-14 at 200nM. Data analysis including Hill plots of the data provided inhibition constants (*see* Bylund & Yamamura, "Methods of receptor binding", *in* Methods in Neurotransmitter Receptor Analysis, Yamamura *et al.*, eds., Raven Press: New York, 1990).

These results are presented in the following Table. The data show that the peptides of the instant invention have a high affinity of binding for somatostatin receptors.

**TABLE II**

| Peptides | Kᵢ(nM) |
|---|---|
| 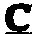 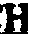 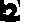 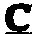 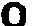 .FYW_{D}KTFC | 0.16 |
| 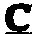 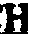 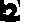 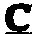 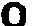 .FFW_{D}KTF.Hhc | 0.41 |
| 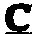 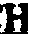 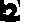 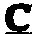 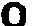 .FFW_{D}KTFC.amide | 0.45 |
| 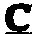 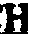 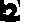 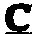 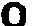 .FFW_{D}KTFC.[BAM] | 1.9 |
| 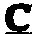 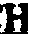 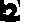 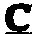 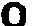 .NFFW_{D}KTFTC | 2.7 |
| 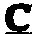 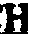 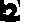 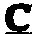 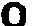 .FFW_{D}KTFC | 4.0 |
| 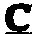 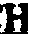 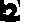 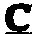 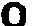 .FFW_{D}KTFCC_{Acm}GC_{Acm}.amide | 7.5 |
| 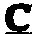 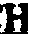 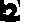 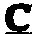 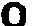 .FFW_{D}KTF.Hcy | 9.8 |

It should be understood that the foregoing disclosure emphasizes certain specific embodiments of the invention and that all modifications or alternatives equivalent thereto are within the spirit and scope of the invention as set forth in the appended claims.

## Claims

1. A pharmaceutical composition comprising a therapeutically effective amount of a somatostatin receptor-binding peptide having the formula: 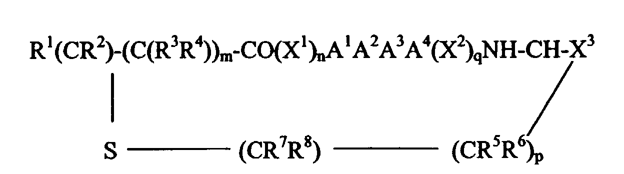 wherein
R¹ and R² are independently H, lower alkyl or substituted alkyl, aryl or substituted aryl;
R³ and R⁴ are each independently H, lower alkyl or substituted alkyl, aryl or substituted aryl, and m is an integer between 0 and 3;
A¹ is 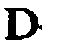 - or 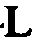 -Phe or 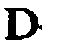 -or 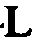 -Tyr or Nal;
A² is 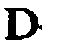 - or 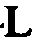 -Trp;
A³ is 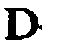 - or 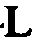 -Lys or Hly, Achxa, Amf, Aec, Apc, Aes or Aps;
A⁴ is Thr, Ser, Val, Phe, Ile, Abu, Nle, Leu, Nva or Aib;
X¹ and X² are each independently a 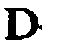 - or 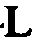 - amino acid, and n and q are independently either 0 or 1;
X³ is H, -COOR⁹; -CH₂OH, -CH₂COOR⁹ or ―CON(R⁹)₂, where each R⁹ is independently H, lower linear or cyclic alkyl, or a peptide having an amino acid sequence of no more than 10 residues;
R⁵ and R⁶ are each independently H or lower alkyl and p is either 0, 1 or 2;
and
R⁷ and R⁸ are independently H, lower alkyl or substituted lower alkyl, or either R⁷ or R⁸ is ―COOH or a derivative thereof;
with the proviso that the somatostatin receptor-binding peptide does not have the general formula: 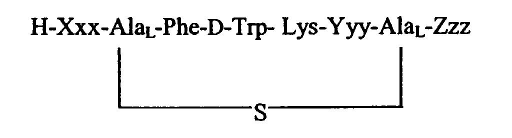 wherein Xxx represents D-Phe, D-β-Nal; Yyy is Thr or Val, and Zzz is TrpNH₂, ThrNH₂, or Thr(ol); and that the somatostatin receptor-binding peptide does not have the general formula:

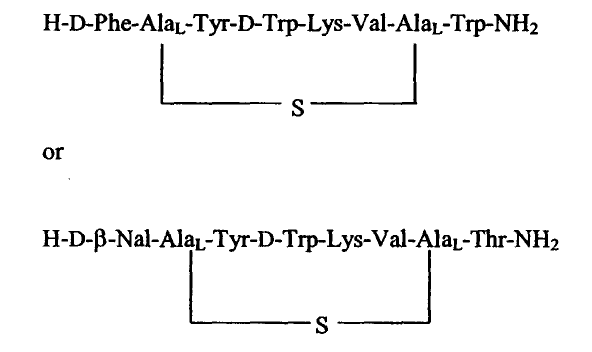

2. The pharmaceutical composition of claim 1, wherein the peptide comprises the following: A¹ is phenylalanine or tyrosine, A² is tryptophan, A³ is lysine and A⁴ is threonine or valine.

3. The pharmaceutical composition of claim 1, wherein the peptide has a formula selected from the group consisting of:
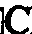 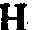 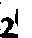 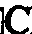 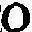 .YW_{D}KTC;
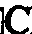 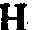 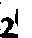 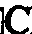 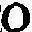 .YW_{D}KTC.amide;
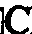 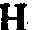 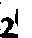 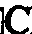 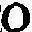 .YW_{D}KTCT;
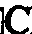 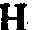 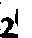 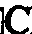 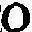 .YW_{D}KTCT(CH₂OH);
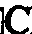 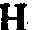 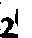 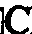 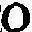 .FFW_{D}KTFC;
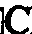 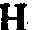 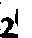 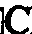 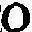 .FFW_{D}KTFC.amide;
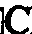 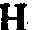 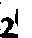 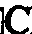 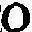 .FW_{D}KTC_{D};
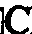 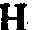 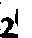 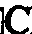 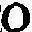 .FW_{D}KT.Hcy;
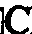 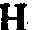 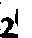 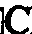 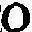 .FW_{D}KT.Hcy.amide;
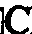 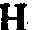 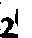 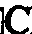 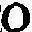 .FW_{D}KT.Pen;
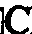 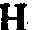 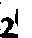 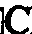 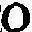 .NFFW_{D}KTFTC;
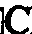 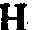 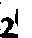 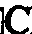 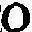 .FFW_{D}KTF.Hcy;
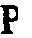 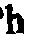 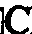 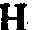 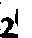 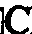 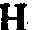 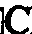 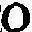 .YW_{D}KTC;
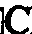 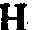 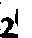 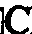 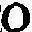 .YW_{D}KT.Hhc;
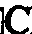 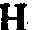 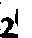 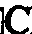 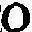 .YW_{D}KT.Hhc.amide;
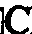 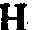 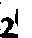 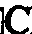 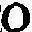 .FFW_{D}KTF.Hhc; and
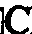 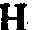 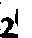 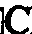 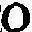 .FYW_{D}KTFC.

4. Use of the composition of any of claims 1 through 3 for the preparation of a medicament for promoting regression of a tumor that expresses somatostatin receptors.
